# FASCICULE DE BREVET EUROPEEN

(11) **EP 0 681 489 B1**
(45) Date de publication et mention de la délivrance du brevet: **05.11.1997**
(21) Numéro de dépôt: 94905752.5
(22) Date de dépôt: 28.01.1994
(51) Int. Cl.: A61M 5/50

(54) **SERINGUE A USAGE LIMITE A UN NOMBRE DETERMINE D'INJECTIONS**
SPRITZE ZUM GEBRAUCH VON EINER ANZAHL LIMITIERTER INJEKTIONEN
SYRINGE FOR ADMINISTERING A GIVEN NUMBER OF INJECTIONS

(30) Priorité: 29.01.1993 FR 9300988; 29.04.1993 FR 9305108
(43) Date de publication de la demande: 15.11.1995
(73) Titulaire: S.C.E.R. SECURINGUE (SOCIETE CIVILE), F-75014 Paris (FR)
(72) Inventeur: LABOUZE, Joseph, F-95230 Soisy-sous-Montmorency (FR)
(74) Mandataire: Laget, Jean-Loup
(86) Numéro de dépôt international: FR9400107
(87) Numéro de publication internationale: WO9416753

(56) Documents cités:
- EP-A- 0 339 954
- EP-A- 0 376 698
- DE-A- 3 833 138

## Description

La présente invention concerne d'une manière générale une seringue et plus particulièrement une seringue à usage limité à un nombre déterminé d'injections, ce nombre pouvant être limité à un.

Depuis de nombreuses années, compte tenu de la prolifération des maladies transmissibles par le sang, l'on recherche assidûment à proposer sur le marché des seringues ne permettant qu'une seule injection qui soit parfaitement non réutilisable.

De très nombreuses propositions ont ainsi été faites mais jusqu'à présent aucune de celles-ci n'a donné totalement satisfaction.

Il est usuellement proposé de mettre en oeuvre des ensembles comportant des crémaillères formées sur le piston de la seringue et coopérant avec un ou des plots solidaires du corps de ladite seringue, de manière à autoriser un mouvement relatif dans un sens de déplacement et à interdire tout mouvement dans le sens opposé de déplacement.

Il a également été proposé dans le EP 0 339 954 une seringue dont le piston porte des plots ou ergots tandis que les crémaillères sont formées sur le corps de la seringue.

Pour l'utilisation de ces seringues, l'on tire le piston vers l'extérieur du corps de la seringue, une crémaillère se déplace ainsi devant un plot autorisant la sortie du piston, ladite crémaillère étant conformée afin d'empêcher tout mouvement de retour dudit piston vers l'intérieur du corps de seringue. Après avoir rempli le corps de seringue au cours de ce mouvement de retrait du piston, il est nécessaire de faire tourner le piston d'un angle prédéterminé afin d'amener la seconde crémaillère, en relation avec le même plot ou un second plot. Dans cette position, le second plot autorise la descente du piston vers le fond du corps de seringue mais interdit tout nouveau mouvement de retrait dudit piston.

Lorsque le piston est ainsi ramené au fond du corps de la seringue, il n'est plus possible de le faire tourner sur lui-même et il est également impossible de le retirer dudit corps du fait de la coopération en blocage de la crémaillère avec le second plot.

L'utilisation d'une seringue de ce type est complexe du fait qu'il est nécessaire de faire tourner le piston sur lui-même d'un angle prédéterminé avant d'éjecter le produit aspiré dans la seringue.

L'on a également proposé dans le DE 38 33 138, qui correspond au préambule de la revendication 1, une seringue à usage unique dont la mise en oeuvre est, pour l'utilisateur, totalement semblable à celle des seringues habituelles. Cette seringue présente un insert monté mobile en rotation dans le corps de seringue. L'insert peut porter soit des ergots coopérant avec des crémaillères formées sur le piston, soit des crémaillères coopérant avec des ergots portés par le piston. L'une des crémaillères est inclinée par rapport à l'axe de la seringue de manière à entraîner l'insert en rotation lors de la sortie du piston et à amener en coopération la crémaillère et l'ergot permettant l'enfoncement du piston.

Ces dispositions présentent l'inconvénient d'être sujettes à des mauvais fonctionnement du fait des forces de frottement intervenant entre le corps de seringue et l'insert et empêchant la rotation dudit insert.

L'on a par ailleurs constaté que les seringues à usage unique proposées présentent usuellement un inconvénient au cours de leur utilisation provenant du fait qu'il est nécessaire de retirer le piston jusqu'au bout de sa course de sortie du corps de seringue avant de pouvoir effectuer les manipulations permettant l'injection et la mise en oeuvre de la seconde crémaillère.

La présente invention tend également à proposer une seringue permettant à l'utilisateur d'effectuer des dosages intermédiaires, c'est-à-dire de ne pas retirer totalement le piston avant l'injection.

Cette seringue permettra ainsi l'injection d'une quantité variable de produit, tout en interdisant après cette injection, toute nouvelle injection.

Par ailleurs, l'on a constaté que la mise en oeuvre de seringues ne permettant qu'une seule injection oblige dans certains cas l'utilisation de deux ou plusieurs seringues pour le même soin.

Il existe en effet un certain nombre de produits dont la mise en oeuvre nécessite le mélange d'un liquide et d'une poudre pour la constitution préalable d'une solution à injecter ensuite.

La présente invention tend alors à proposer une seringue qui, suivant sa constitution, permettra un nombre déterminé d'injections, une pour des utilisations classiques ou deux, ou plus, pour des utilisations spéciales lors de soins ou lors d'analyses en laboratoire.

La présente invention a pour objet une seringue à usage limité à un nombre déterminé d'injections formée d'un corps dans lequel se déplace un piston, du genre comportant au moins deux crémaillères portées par le corps, l'une de ces crémaillères autorisant la sortie du piston et l'autre son retour vers le fond du corps de seringue, et deux ergots portés par le piston et entraînés en translation par celui-ci, chacune desdites crémaillères pouvant avoir vis-à-vis d'un ergot un déplacement relatif dans une seule direction, caractérisée en ce que :
- lesdites crémaillères sont fixées sur le corps,
- lesdites crémaillères sont séparées par une nervure,
- l'un des ergots est destiné à se mettre en butée contre les languettes d'une crémaillère pour bloquer tout mouvement inverse du piston au cours du remplissage de la seringue, l'autre ergot est destiné à se mettre en butée contre les languettes de l'autre crémaillère pour bloquer tout mouvement de retrait du piston alors qu'il est repoussé vers le fond du corps de seringue,
- lesdits ergots sont portés par un étrier chevauchant le piston et mobile en rotation autour de l'axe dudit piston de manière à être entraînés en translation par le piston tout en étant libres en rotation par rapport à celui-ci,
- une pointe incurvée ou languette oblique est formée à l'extrémité de la nervure afin d'amener les ergots en regard de la crémaillère autorisant le retour du piston vers le fond du corps de seringue.

La seringue selon l'invention, au moins dans sa forme préferée, est encore remarquable en ce que :
- la crémaillère autorisant la sortie du piston comporte au moins une languette oblique inclinée par rapport à l'axe de la seringue de façon à autoriser son franchissement par les ergots de l'étrier lors de la sortie du piston, et à imposer à l'étrier, lors de la rentrée du piston, un mouvement de rotation pour placer les ergots de l'étrier en regard des languettes de la deuxième crémaillère de telle sorte que, après franchissement par les ergots de chacune des languettes obliques de la première crémaillère, la rentrée du piston soit autorisée,
- elle comporte plusieurs couples de crémaillères alternées, la première crémaillère autorisant la sortie du piston du corps de seringue, et la dernière le retour du piston vers le fond du corps de seringue, et des languettes obliques situées à l'extrémité de chaque crémaillère hormis la dernière pour assurer la rotation de l'étrier de manière que les ergots coopèrent avec la crémaillère suivante à chaque inversion du sens de déplacement du piston,
- les crémaillères d'ordre pair sont à languettes transversales autorisant un déplacement longitudinal du piston en interdisant le mouvement longitudinal inverse du piston, et l'une au moins des crémaillères d'ordre impair est à languettes obliques inclinées par rapport à l'axe de la seringue de manière à autoriser le déplacement longitudinal du piston dans une direction et à interdire un retour direct du piston vers le fond du corps de seringue, lesdites languettes obliques forçant l'ergot coopérant avec elles lors du mouvement de retour du piston vers le fond du corps de seringue à avoir un mouvement circonférentiel l'écartant de l'axe de la crémaillère,
- l'étrier a une section transversale globalement en U et porte lesdits ergots sur sa face externe dans la portion arrondie du U,
- les extrémités dudit U sont bordées de nervures sur leurs surfaces extérieures,
- l'un au moins des deux ergots est effilé en direction de l'extrémité axiale de l'étrier,
- les crémaillères sont formées sur un insert fixé au corps de seringue,
- l'insert est formé d'un corps demi-cylindrique terminé par une plaque d'extrémité qui lui est perpendiculaire,
- le corps de l'insert est séparé, par une nervure longitudinale disposée sur sa paroi interne se terminant par une pointe incurvée s'étendant dans un crevé, en deux parties portant chacune une série de languettes dont les extrémités dépassent de la paroi interne en direction de l'axe afin de confèrer audit corps une section longitudinale en forme de crémaillères,
- la pointe incurvée a son extrémité disposée longitudinalement à l'avant des languettes formant la crémaillère,
- le piston comporte deux portions reliées par une tige axiale cylindrique,
- ladite tige axiale cylindrique est séparée en deux parties par une liaison fragile,
- le diamètre de ladite tige axiale et sa longueur sont tels que l'étrier puisse être positionné sur elle de manière à la chevaucher,
- la distance séparant axialement la face interne de la plaque d'extrémité de l'insert de l'extrémité de la pointe incurvée est supérieure à la longueur de l'étrier de sorte que lorsque le mouvement de sortie du piston est terminé, l'étrier soit totalement disposé entre la plaque d'extrémité et ladite pointe s'étende axialement dans le prolongement des ergots, et que, lors du mouvement de retour du piston vers l'intérieur du corps de la seringue, l'ergot entre en contact avec ladite pointe incurvée et entraîne l'étrier en rotation pour amener les ergots dans l'axe des languettes formant la crémaillère, sans que le piston soit entraîné en rotation.
- elle porte une aiguille double constituée de deux aiguilles insérées l'une dans l'autre, une aiguille fine interne soudée à l'extrémité du corps de seringue et une aiguille amovible de diamètre supérieur positionnée autour de ladite aiguille fine de façon à pratiquer l'injection au patient avec l'aiguille fine.

L'invention sera mieux comprise grâce à la description qui va suivre donnée à titre d'exemple non limitatif en référence aux dessins annexés dans lesquels :
- la figure 1 est une vue, partiellement en coupe longitudinale de la seringue selon l'invention,
- la figure 2 est une vue en coupe transversale suivant la ligne II-II de la figure 1 pour une seringue ne permettant qu'une injection,
- la figure 3 est une vue en perspective de l'insert,
- la figure 4 est une vue en perspective de l'étrier,
- la figure 5 est une vue partiellement en coupe longitudinale du piston portant l'étrier et positionné dans l'insert,
- la figure 6 représente la seringue après utilisation.
- la figure 7 est une vue développée du corps de l'insert d'une seringue selon l'invention permettant le dosage du produit injecté,
- la figure 8 est une vue partiellement en coupe longitudinale du piston portant l'étrier positionné dans l'insert,
- la figure 9 est une vue semblable à la figure 2 pour une seringue permettant deux injections,
- la figure 10 est une vue développée du corps de l'insert pour une seringue permettant deux injections,
- la figure 11 montre schématiquement un mode de réalisation de la seringue.

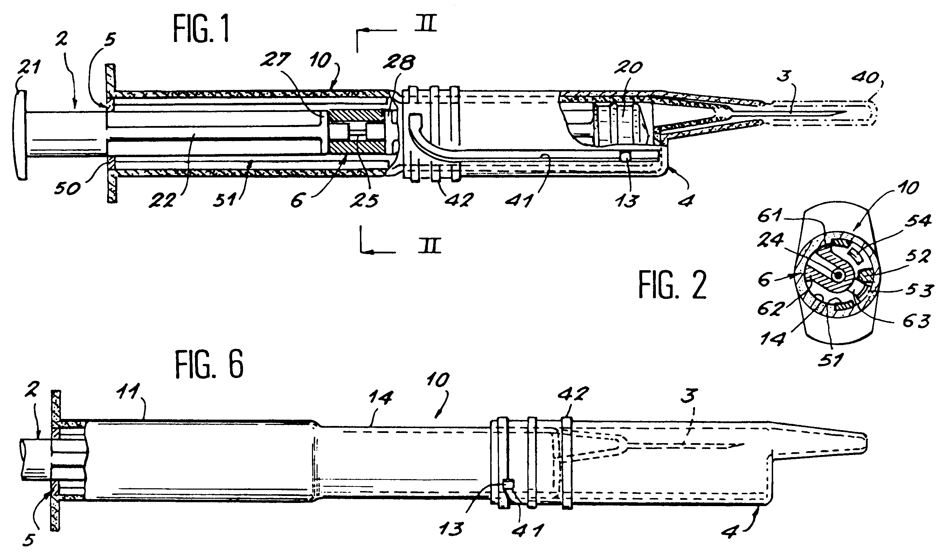

L'on voit sur la figure 1 que la seringue selon l'invention comporte de manière connue en soi, un corps de seringue 10 à l'intérieur duquel est disposé un piston 2 présentant une garniture d'étanchéité 20 à son extrémité disposée dans le corps 10 et une plaque de poussée 21 à son extrémité disposée à l'extérieur du corps 10.

Ledit corps 10 porte également une aiguille 3 et un capuchon 4 de protection de cette aiguille.

De manière connue en soi, ledit capuchon 4 comporte une portion d'extrémité 40 détachable pour découvrir l'aiguille 3 en vue de l'utilisation de la seringue.

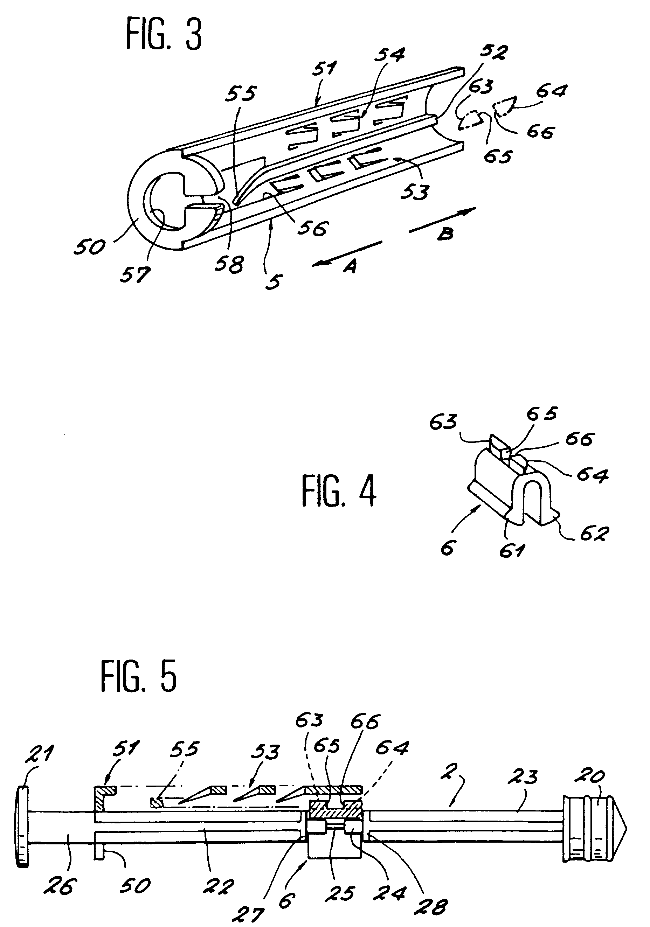

Comme on peut le voir sur la figure 3, l'insert 5 est formé, dans l'exemple représenté, d'un corps 51 ayant la forme d'un demi-cylindre terminé par une plaque d'extrémité 50 qui lui est perpendiculaire. Un tel corps demi-cylindrique est préféré pour réduire la quantité de matière utilisée ainsi que pour faciliter la réalisation dudit insert par moulage.

La dimension circonférentielle dudit corps 51 peut être modifiée et dépend comme on pourra le comprendre à la lecture de la description qui va suivre du nombre d'injections que l'on veut autoriser ainsi que du diamètre de la seringue.

Le corps 51 est séparé en deux parties par une nervure 52 longitudinale disposée sur sa paroi interne et se terminant par une pointe incurvée 55 s'étendant dans un crevé 56. Cette pointe incurvée 55 est, comme représenté à la figure 5, disposée à distance de la paroi externe de l'insert.

Chaque partie du corps 51 de l'insert porte une série de languettes dont les extrémités dépassent de la paroi interne en direction de l'axe. Chaque série de languettes confère au corps de l'insert une section longitudinale en forme de crémaillère. La crémaillère 53 est formée de languettes disposées avec leurs faces d'extrémité dirigées vers la plaque d'extrémité 50, tandis que la crémaillère 54 est formée de languettes orientées en direction opposée.

La pointe incurvée 55 est telle que son extrémité soit disposée longitudinalement à l'avant des languettes formant la crémaillère 53.

La plaque d'extrémité 50 présente une ouverture 57 débouchant, dans l'exemple de réalisation représenté, sur le pourtour de ladite plaque 50 par une rainure 58.

A la figure 3, l'ouverture 57 est arrondie, le diamètre de l'arrondi étant égal à celui de la tige du piston 2.

L'étrier 6 mis en oeuvre dans la seringue selon l'invention présente, comme on peut le voir sur la figure 4, une section transversale globalement en U. Les extrémités dudit U sont bordées sur leurs surfaces extérieures de nervures 61 et 62.

L'étrier 6 présente encore des ergots 63, 64 disposés longitudinalement sur sa face externe dans la portion arrondie du U.

Les deux ergots 63 et 64 sont effilés en direction des extrémités axiales de l'étrier 6.

Lesdits ergots 63 et 64 peuvent être tels que représentés à la figure 4 ou avoir une forme ressemblant à une ogive.

Comme il sera exposé plus loin, l'un seul de ces ergots doit impérativement être ainsi effilé, mais l'on préfère prévoir les deux ergots effilés pour des raisons pratiques de montage de la seringue selon l'invention. Les extrémités 65, 66 en vis à vis des ergots sont constituées de faces planes perpendiculaires à l'axe de l'étrier 6.

La constitution du piston 2 de la seringue selon l'invention va maintenant être décrite en liaison avec la figure 5.

De manière usuelle, les pistons de seringues présentent une tige à section en croix formées de quatre nervures positionnées perpendiculairement les unes aux autres.

Cette conformation usuelle est reprise, dans la forme de réalisation représentée, pour deux portions 22 et 23 de la tige de piston 2, toute autre forme de section pouvant bien entendu être utilisée. Une portion 26 cylindrique est, dans l'exemple représenté, formée entre la plaque de poussée 21 et la première portion en croix 22.

Lesdites portions 22 et 23 en croix sont reliées l'une à l'autre par une tige axiale cylindrique de plus petit diamètre 24 pouvant être, comme représenté, séparée en deux parties par une liaison fragile 25 de très faible diamètre.

Des plaques circulaires 27, 28 perpendiculaires à l'axe du piston sont disposées entre chacune des deux portions en croix 22, 23 et la tige axiale 24.

Le diamètre de ladite tige axiale 24 et sa longueur sont tels que l'étrier 6 puisse être positionné sur elle de manière à la chevaucher en étant libre en rotation autour de l'axe du piston tout en étant maintenu axialement par les plaques circulaires 27, 28.

L'insert 5 est quant à lui positionné autour de la partie en croix 22 en introduisant trois nervures de celle-ci dans l'ouverture 57 tandis que la quatrième nervure est dans la rainure 58 de la plaque d'extrémité 50.

Cette mise en place de la tige de piston dans l'ouverture de la plaque d'extrémité de l'insert 5 peut être faite en utilisant l'élasticité des matériaux choisis pour la réalisation par moulage de l'insert 5.

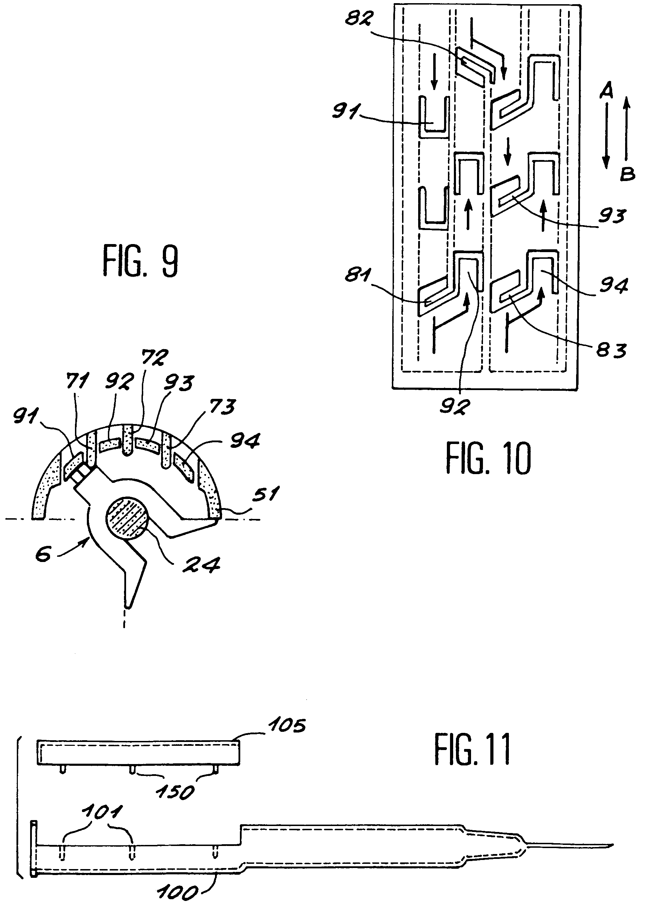

Il est également possible de prévoir dans deux nervures diamétralement opposées de la tige de piston des entailles afin de former une portion diamétrale plane 29 telle que celle visible à la figure 8. Le piston 2 est dans ce cas positionné dans l'insert 5 en glissant ladite portion diamétrale plane 29 dans la rainure 58.

Lors du montage, l'insert 5 est positionné autour de la tige de piston portant l'étrier 6 de manière telle que la nervure 61 soit en appui contre le bord du corps 51 dudit insert tandis que les ergots 63, 64 sont axialement alignés avec les languettes formant la crémaillère 53. Cette position est représentée à la figure 2 montrant la seringue avant utilisation.

Comme l'on peut également le voir sur cette figure 2, l'on conforme la surface interne du corps de seringue avec un diamètre un peu inférieur sur une moitié 14 de celle-ci afin de faciliter la mise en place de l'insert 5 dans ce corps de seringue. Celui-ci peut ainsi être disposé en butée contre les parois longitudinales bordant cette moitié 14 de plus faible diamètre. L'insert est ensuite fixé au corps de seringue par exemple en solidarisant la plaque d'extrémité 50 par collage, soudure ...

Le piston 2 muni de l'étrier 6 est alors déplaçable à l'intérieur de l'insert 5 fixé au corps 10 de seringue.

Comme à l'habitude, pour l'utilisation d'une seringue, l'on tire dans un premier temps le piston 2 vers l'extérieur du corps de seringue afin de remplir ledit corps 10.

Au cours de ce mouvement, les ergots 63 et 64 de l'étrier 6 glissent le long des languettes formant la première crémaillère 53 en se déplaçant dans le sens A représenté à la figure 3. Tout mouvement inverse du piston 2 au cours du remplissage est bloqué par la mise en butée de l'extrémité plane 65 de l'ergot 63 contre l'extrémité de l'une des languettes formant ladite crémaillère 53.

Le mouvement de sortie du piston 2 se poursuit jusqu'à ce que l'étrier 6 arrive en contact avec la face interne de la plaque d'extrémité 50 de l'insert 5, la pointe 55 s'effaçant latéralement vers l'extérieur dans l'espace libre situé au droit de la paroi de l'insert lors du passage des ergots contre elle.

La distance séparant axialement la face interne de la plaque d'extrémité 50 de l'extrémité de la pointe incurvée 55 est prévue un peu supérieure à la longueur de l'étrier 6.

Ainsi, lorsque le piston 2 est en bout de course de sortie, l'étrier 6 est totalement disposé entre la plaque d'extrémité 50 et ladite pointe 55.

La pointe 55 étant incurvée de manière telle que son extrémité soit disposée à l'avant des languettes formant la crémaillère 53, celle-ci s'étend axialement dans le prolongement des ergots 63 et 64.

Par cette disposition, lorsque l'utilisateur repousse le piston 2 vers l'intérieur du corps 10 de la seringue afin de la vider de son contenu, l'ergot 64 entre en contact avec la pointe incurvée 55.

Pour une meilleure compréhension de l'invention, l'on a représenté les ergots 63 et 64 en pointillés sur la figure 3.

L'ergot 64 étant effilé, il peut glisser le long de la pointe incurvée 55 ce qui entraîne l'étrier 6 en rotation autour de la tige 24 jusqu'à prendre une position symétrique, par rapport au plan diamétral passant par la nervure 52, de la position dans laquelle il est représenté à la figure 2.

Comme déjà suggéré, l'on comprend que seul l'ergot 64 doit être effilé pour coopérer avec la pointe incurvée 55. De manière préférée, l'on forme les deux ergots de la même façon ce qui évite de rechercher le sens de positionnement de l'étrier 6 sur le piston 2.

Dans cette nouvelle position, la nervure 62 est en butée contre la bordure du corps 51 tandis que les ergots 64 et 63 sont disposés dans l'axe des languettes formant la crémaillère 54.

Le piston 2 peut alors être repoussé dans la direction B tandis que tout mouvement de retrait dans la direction A est bloqué par l'appui de la face 66 de l'ergot 64 contre l'extrémité de l'une des languettes formant la crémaillère 54.

La longueur séparant deux extrémités tournées dans le même sens des ergots portés par l'étrier est prévue largement différente de celle séparant les extrémités de deux languettes successives afin d'assurer que seule l'une des faces 65 ou 66 entre en contact avec l'extrémité d'une languette sans risquer que l'ergot non concerné agisse en interférence. De manière préférée, l'étrier est prévu plus court que la longueur séparant les extrémités de deux languettes consécutives.

Comme on aura pu le comprendre à la lecture de la description qui vient d'être faite, la manipulation de la seringue selon l'invention est, pour l'utilisateur, totalement identique à celle des seringues classiques. Le passage de l'une à l'autre des crémaillères est fait de manière invisible pour l'utilisateur par l'entraînement en rotation de l'étrier.

Ainsi, la mise en oeuvre de crémaillères fixées sur le corps de seringue tandis que les ergots destinés à coopérer avec ces crémaillères sont portés par le piston de manière à être entrainés en translation et libres en rotation par rapport à celui-ci permet de fournir une seringue à usage unique de manipulation tout à fait simple et sûre en évitant de plus tout problème de coincement du à des frottements excessifs.

Quelle que soit la position du piston par rapport au corps 10 de seringue au cours du mouvement tendant à le repousser vers le fond dudit corps, tout mouvement de retrait est, comme déjà décrit, bloqué. Si l'utilisateur tente alors de surmonter ce blocage, la liaison fragile 25 se brise rendant ainsi la seringue définitivement inutilisable.

La seringue selon l'invention comporte d'autres dispositions qui vont maintenant être décrites.

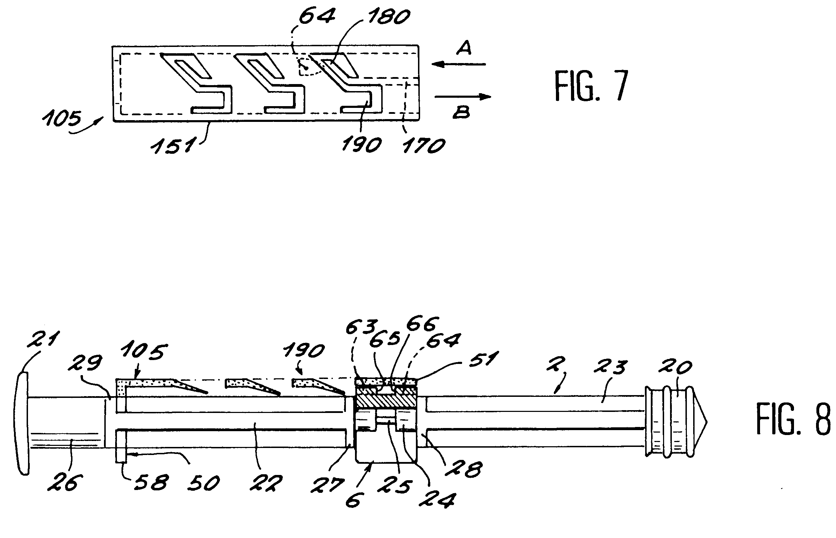

Comme on peut le voir sur la figure 6, le corps 10 de la seringue est formé de deux cylindres 11 et 12 mis bout à bout.

Le cylindre 11 est de diamètre un peu supérieur à celui du cylindre 12. Cette différence de diamètre correspond globalement à l'épaisseur du corps 51 de l'insert 5.

La garniture 20 du piston 2 est disposée dans le cylindre 12 et adaptée à coulisser avec étanchéité le long de la paroi interne de celui-ci.

Le cylindre 11 est de longueur sensiblement égale à celle de l'insert et celui-ci est disposé contre la paroi interne dudit cylindre 11.

Lesdits cylindres 11 et 12 sont de longueurs sensiblement égales, l'étrier 6 et la garniture 20 du piston étant également de même longueur.

Ainsi, lorsque le piston est retiré jusqu'à ce que l'étrier soit en contact avec la face d'extrémité de l'insert, la garniture d'étanchéité parcourt la longueur du cylindre 12 qui représente le volume utile d'utilisation de la seringue.

Bien entendu, il faut prendre garde, lors de la définition des dimensions et des tolérances de fabrication des différentes pièces, que la garniture 20 ne puisse en aucun cas arriver en butée contre l'extrémité du cylindre 11, ou de l'insert 5, avant que les ergots de l'étrier aient dépassé la pointe incurvée 55.

En effet, si une telle disposition se produisait, la seringue serait irrémédiablement inutilisable.

La différence de diamètre entre les cylindres 11 et 12 n'a aucune influence sur le fonctionnement de la seringue selon l'invention et pourrait, sans sortir du cadre de la présente invention, ne pas être reprise.

Cette différence n'est prévue que pour procurer une seringue portant un capuchon 4 de protection de l'aiguille 3 d'aspect extérieur uniforme.

En effet, comme représenté à la figure 1, ledit capuchon de protection 4 est, avant l'utilisation de la seringue, disposé autour du cylindre 12. L'épaisseur dudit capuchon 4 étant identique à celle de l'insert 5, l'on procure une seringue de diamètre externe sensiblement constant, les nervures de préhension 42 mises à part.

Le capuchon de protection 4 comporte une lumière 41 disposée axialement et se terminant par une extrémité arquée.

Un ergot 13 disposé sur la surface externe du corps de seringue est destiné à être positionné dans ladite lumière afin de coopérer avec elle.

La seringue est fournie dans la disposition représentée à la figure 1 avec l'embout 40 solidaire du capuchon de protection.

L'utilisateur détache alors ledit embout 40, par exemple en lui imprimant un mouvement de rotation, afin de découvrir l'aiguille 3.

Comme à l'habitude, il tire sur le piston pour remplir la seringue puis repousse ledit piston. Au cours de ce mouvement de va et vient l'étrier tourne comme précédemment décrit afin de changer la crémaillère utilisée et d'interdire tout nouveau retrait du piston.

Lorsque l'utilisateur a terminé, il tire le capuchon de manière à l'amener dans la position représentée à la figure 6 pour laquelle il recouvre l'aiguille souillée et jette la seringue.

Lors du retrait dudit capuchon, l'ergot 13 est amené dans la partie d'extrémité de la lumière 41. Un mouvement de rotation est de ce fait nécessaire pour ramener le capuchon autour du corps de seringue, ce qui permet d'éviter tout découvrement intempestif de l'aiguille lors de manipulations postérieures de la seringue.

L'on peut prévoir, moyennant quelques modifications mineures d'adapter une seringue selon la présente invention afin que celle-ci puisse être commercialisée préremplie, par exemple pour des produits de vaccination.

Afin d'assurer une bonne conservation des produits, il est nécessaire qu'ils soient conditionnés dans des matériaux neutres tels que du verre. A cet effet, l'on propose de mettre en oeuvre une cuve en verre présentant une aiguille disposée à travers son fond et de former le corps de la seringue en surmoulant ladite cuve avec une matière plastique.

La figure 7 représente schématiquement en vue développée le corps 151 de l'insert 105 mis en oeuvre pour une seringue selon l'invention permettant une seule injection avec possibilité d'effectuer un dosage du produit que l'on injecte.

Ce corps 151 présente deux crémaillères 190 et 180, l'une à languettes transversales et l'autre comportant au moins une languette oblique séparées par une nervure longitudinale 170 discontinue.

Ces crémaillères sont disposées de manière à ce que les extrémités des languettes les constituant dépassent vers l'intérieur du corps de l'insert afin de coopérer avec les ergots 63 et 64 portés par l'étrier 6.

La crémaillère 180 comporte dans l'exemple représenté plusieurs languettes obliques c'est-à-dire inclinées par rapport à l'axe de la seringue de manière à autoriser la sortie du piston dans la direction A et à interdire longitudinalement un retour direct du piston vers le fond du corps de seringue. Les languettes obliques de cette crémaillère forcent l'ergot 64 coopérant avec elles lors du mouvement de retour du piston vers le fond du corps de seringue à avoir un mouvement circonférentiel l'écartant de l'axe de la crémaillère.

Les languettes de la crémaillère 180 sont donc disposées de manière à obliger l'étrier 6 portant l'ergot 64 à tourner autour de l'axe du piston de manière à amener ledit ergot 64 en coopération avec les languettes formant la crémaillère 190.

La crémaillère 190 est à languettes transversales de manière à autoriser un déplacement longitudinal du piston dans la direction B dirigée vers le fond de la seringue en interdisant tout mouvement longitudinal de sortie dudit piston par butée contre l'extrémité plane 66 de l'ergot 64.

Par une telle disposition, l'utilisateur de la seringue tire le piston vers l'extérieur du corps de seringue dans la direction A jusqu'à ce qu'il ait aspiré le volume utile de produit.

A tout moment, dès lors que l'ergot 64 a dépassé au moins l'une des languettes obliques de la crémaillère 180, il peut repousser le piston afin de procéder à l'injection. Lors de ce mouvement, l'ergot butte contre la languette située au-dessous de la position jusqu'à laquelle il a été tiré. La languette le dévie alors, ce qui fait tourner l'étrier jusqu'à ce que ledit ergot vienne en coopération avec la crémaillère 190.

Le mouvement dans la direction B se poursuit alors jusqu'à ce que le piston soit au fond du corps de la seringue.

La crémaillère 190 autorisant le mouvement dans la direction B mais interdisant tout mouvement en sens inverse, il est alors totalement impossible de réutiliser la seringue pour une seconde injection.

Les languettes obliques de la crémaillère 180 peuvent être prévues espacées comme représenté sur le dessin puisqu'elles ont pour mission de faire dévier l'étrier en direction de la crémaillère 190 et non de bloquer le mouvement du piston vers le fond comme dans les seringues proposées jusqu'à maintenant.

Cet espace peut être mis à profit par l'utilisateur de la seringue pour faire effectuer un mouvement de va-et-vient au piston permettant d'évacuer des bulles du liquide aspiré.

Bien entendu cet espace est prévu assez petit pour ne pas rendre possible une injection de produit lors d'un tel va-et-vient.

Il faut noter que ce mouvement de va-et-vient n'est possible qu'entre la position pour laquelle l'ergot 64 touche une des languettes et la position pour laquelle l'ergot 63 touche la languette suivante. En effet, lorsque l'ergot 63 a dépassé la languette suivante, le mouvement de retour du piston vers le fond du corps de seringue est interdit par coincement de ladite languette entre les deux ergots 63 et 64 tant que l'ergot 64 n'a pas lui aussi dépassé ladite languette.

Les figures 9 et 10 représentent une seringue selon l'invention autorisant deux injections.

Il est nécessaire pour chaque injection, de disposer d'un couple de crémaillères et d'un étrier mobile en rotation sur le piston afin de pouvoir se positionner devant la seconde crémaillère autorisant le retour du piston vers le fond du corps de seringue.

La seringue permettant deux injections est alors prévue avec deux couples de crémaillères disposés côte à côte.

Une languette inclinée est disposée à l'extrémité de la seconde crémaillère du couple permettant la première injection afin d'entraîner l'étrier en rotation de manière à ce que les ergots qu'il porte viennent en coopération avec la première crémaillère du second couple.

Dans ce cas, le corps 251 de l'insert est séparé en quatre parties par des nervures longitudinales 71, 72 et 73 disposées sur sa paroi interne et se terminant chacune par une languette oblique respectivement 81, 82 et 83.

Chaque partie du corps 251 de l'insert porte une série de languettes dont les extrémités dépassent de la paroi interne en direction de l'axe afin de former des crémaillères. Les crémaillères 91 et 93 sont formées de languettes disposées avec leurs faces d'extrémité dirigées vers la plaque d'extrémité de l'insert, tandis que les crémaillères 92 et 94 sont formées de languettes orientées en direction opposée.

Les pointes incurvées 81, 82 et 83 sont respectivement telles que leurs extrémités soient disposées longitudinalement à l'avant des languettes formant les crémaillères 91, 92 et 93.

Dans l'exemple représenté, la crémaillère 93 est une crémaillère à languettes obliques obligeant l'étrier à tourner lors de la coopération de l'ergot 64 avec chacune des languettes. La languette oblique 83 est alors constituée par la dernière languette de ladite crémaillère 93.

Lors du montage, l'insert est positionné autour de la tige de piston portant l'étrier 6 de manière telle que les ergots 63, 64 soient axialement alignés avec les languettes formant la première crémaillère 91.

Comme à l'habitude, pour l'utilisation d'une seringue, l'on tire dans un premier temps le piston 2 vers l'extérieur du corps de seringue afin de remplir ledit corps 10.

Au cours de ce mouvement, les ergots 63 et 64 de l'étrier 6 glissent le long des languettes formant la première crémaillère 91 en se déplaçant dans le sens A représenté à la figure 10. Tout mouvement inverse du piston 2 au cours du remplissage est bloqué par la mise en butée de l'extrémité plane 65 de l'ergot 63 contre l'extrémité de l'une des languettes transversales formant ladite première crémaillère 91.

Le mouvement de sortie du piston 2 se poursuit jusqu'à ce que l'étrier 6 arrive en contact avec la face interne de la plaque d'extrémité de l'insert, la pointe 81 s'effaçant lors du passage des ergots. Dans cette position atteinte en fin de course, la languette oblique 81 s'étend axialement dans le prolongement des ergots 63 et 64.

Lorsque l'utilisateur repousse le piston 2 vers l'intérieur du corps 10 de la seringue afin de la vider de son contenu, l'ergot 64 entre en contact avec la languette oblique 81.

L'ergot 64 effilé, glisse alors le long de la languette oblique 81 ce qui entraîne l'étrier 6 en rotation autour de la tige 24 jusqu'à ce que les ergots 63, 64 soient axialement en face des languettes formant la deuxième crémaillère 92.

Le piston 2 peut alors être repoussé dans la direction B tandis que tout mouvement de retrait dans la direction A est bloqué par l'appui de la face 66 de l'ergot 64 contre l'extrémité de l'une des languettes transversales formant la deuxième crémaillère 92.

Ce mouvement de sortie et de rentrée du piston 2 permet d'effectuer la première injection.

Lorsque le piston 2 est de nouveau au fond du corps 10 de la seringue après la première injection, les ergots 63 et 64 sont axialement alignés avec la languette oblique 82. Lorsque l'on tire à nouveau le piston 2 vers l'extérieur pour aspirer le produit destiné à la seconde injection, l'ergot 63 bute contre la languette oblique 82 ce qui entraîne à nouveau l'étrier 6 en rotation autour de la tige 24.

Ce mouvement de rotation de l'étrier amène les ergots 63, 64 dans le prolongement axial de la crémaillère 93.

Le piston peut alors être à nouveau retiré dans la direction de la flèche A pour remplir la seringue de la quantité désirée de produit, puis être repoussé, l'étrier 6 étant entraîné en rotation par l'une des languettes obliques de la crémaillère 93 de manière à amener les ergots en coopération avec la quatrième crémaillère 94.

Quelle que soit la position du piston par rapport au corps 10 de seringue au cours d'un mouvement tendant à le repousser vers le fond dudit corps, tout mouvement de retrait est bloqué par la coopération de la face 66 de l'ergot 64 avec l'extrémité des languettes formant la crémaillère 92 ou la crémaillère 94. Si l'utilisateur tente alors de surmonter ce blocage, la liaison fragile 25 se brise rendant ainsi la seringue définitivement inutilisable.

Dans l'exemple représenté, seule la seconde injection peut être effectuée avec un volume choisi de produit. Cet exemple correspond à un grand nombre de traitements pour lesquels le produit à injecter est proposé sous la forme de deux constituants à mélanger. Dans ces cas, il faut généralement mélanger la totalité du liquide à la totalité de la poudre même si la quantité à injecter au patient est bien inférieure.

Il est bien entendu possible de proposer une seringue autorisant également le dosage du produit liquide à utiliser pour le mélange en remplaçant la crémaillère 91 à languettes transversales par une crémaillère à languettes obliques.

Lorsque la seringue est constituée afin de permettre deux injections, l'on peut prévoir une aiguille double constituée de deux aiguilles insérées l'une dans l'autre, une aiguille fine interne soudée à l'extrémité du corps de seringue et une aiguille amovible de diamètre supérieur positionnée autour de ladite aiguille fine.

Ainsi, l'on perce le bouchon du flacon contenant le liquide avec l'aiguille de grand diamètre, l'on fait le mélange à injecter, puis l'on enlève l'aiguille de grand diamètre afin de découvrir et utiliser l'aiguille fine pour injecter le produit au patient.

La présente invention a été décrite en liaison avec des dessins montrant une seringue permettant deux injections, il est bien entendu possible d'adapter l'insert afin de prévoir des crémaillères et pointes incurvées supplémentaires si la nécessité de fournir des seringues permettant un plus grand nombre d'injections se fait sentir.

Le nombre d'injections pouvant être prévu dépend également de la dimension circonférentielle que peut avoir l'insert et donc du diamètre de la seringue.

Le montage d'une seringue selon l'invention a été décrit précédemment en positionnant un sous-ensemble constitué de l'insert, du piston et de l'étrier dans le corps de seringue et en solidarisant l'insert audit corps de seringue.

En variante, comme représenté à la figure 11, l'insert 105 est conçu sous la forme d'une portion du corps 100 de seringue et est rapporté et fixé sur ledit corps par tout moyen connu, soudure, colle, ...

Le positionnement précis de l'insert est assuré par la coopération de pions 150 avec des ouvertures 101.

L'insert est fixé sur le corps après la mise en place du piston portant l'étrier dans ledit corps.

## Revendications

1. Seringue à usage limité à un nombre déterminé d'injections formée d'un corps (10) dans lequel se déplace un piston (2), du genre comportant au moins deux crémaillères (53, 54 ; 180, 190 ; 91, 92 ; 93, 94) formées de languettes et portées par le corps (10), l'une (53, 180, 91, 93) de ces crémaillères autorisant la sortie du piston (2) et l'autre (54, 190, 92, 94) son retour vers le fond du corps (1) de seringue, et deux ergots (63, 64) portés par le piston (2) et entraînés en translation par celui-ci, chacune desdites crémaillères pouvant avoir vis-à-vis d'un ergot un déplacement relatif dans une seule direction, caractérisée en ce que :
- lesdites crémaillères (53, 54 ; 180, 190 ; 91, 92 ; 93, 94) sont fixées sur le corps (10),
- lesdites crémaillères (53, 54 ; 180, 190 ; 91, 92 ; 93, 94) sont séparées par une nervure (52, 170, 71, 72, 73),
- l'un (63) des ergots est destiné à se mettre en butée contre les languettes d'une crémaillère (53) pour bloquer tout mouvement inverse du piston (2) au cours du remplissage de la seringue, l'autre ergot (64) est destiné à se mettre en butée contre les languettes de l'autre crémaillère (54) pour bloquer tout mouvement de retrait du piston alors qu'il est repoussé vers le fond du corps de seringue,
- lesdits ergots (63, 64) sont portés par un étrier (6) chevauchant le piston (2) et mobile en rotation autour de l'axe dudit piston (2) de manière à être entraînés en translation par le piston (2) tout en étant libres en rotation par rapport à celui-ci,
- une pointe incurvée ou languette oblique (55, 81, 83) est formée à l'extrémité de la nervure (52, 71, 73) afin d'amener les ergots (63, 64) en regard de la crémaillère autorisant le retour du piston (2) vers le fond du corps de seringue.

2. Seringue selon la revendication 1, caractérisée en ce que la crémaillère (180) autorisant la sortie du piston (2) comporte au moins une languette oblique par rapport à l'axe de la seringue de façon à autoriser son franchissement par les ergots (63, 64) de l'étrier (6) lors de la sortie du piston et à imposer à l'étrier (6), lors de la rentrée du piston, un mouvement de rotation pour placer les ergots (63, 64) de l'étrier (6) en regard des languettes de la deuxième crémaillère (190) de telle sorte que, après franchissement de chacune des languettes obliques de la crémaillère, la rentrée du piston soit autorisée.

3. Seringue selon la revendication 1, caractérisée en ce qu'elle comporte plusieurs couples de crémaillères alternées (91, 92 ; 93, 94), la première crémaillère (91) autorisant la sortie du piston (2) du corps de seringue, et la dernière (94) le retour du piston (2) vers le fond du corps de seringue, et des languettes obliques (81, 82, 83) situées à l'extrémité de chaque crémaillère hormis la dernière pour assurer la rotation de l'étrier (6) de manière que les ergots (63, 64) coopèrent avec la crémaillère suivante à chaque inversion du sens de déplacement du piston.

4. Seringue selon la revendication 3, caractérisée en ce que les crémaillères (54; 190; 92; 94) d'ordre pair sont à languettes transversales autorisant un déplacement longitudinal du piston (2) en interdisant le mouvement longitudinal inverse dudit piston (2), et l'une au moins des crémaillères (53; 180; 91; 93) d'ordre impair est à languettes obliques inclinées par rapport à l'axe de la seringue de manière à autoriser le déplacement longitudinal du piston (2) dans une direction et à interdire un retour direct du piston (2) vers le fond du corps de seringue, lesdites languettes obliques forçant l'ergot (64) coopérant avec elles lors du mouvement de retour du piston vers le fond du corps de seringue à avoir un mouvement circonférentiel l'écartant de l'axe de la crémaillère.

5. Seringue selon la revendication 1, caractérisée en ce que l'étrier (6) a une section transversale globalement en U et porte lesdits ergots (63, 64) sur sa face externe dans la portion arrondie du U.

6. Seringue selon la revendication 5, caractérisée en ce que les extrémités dudit U sont bordées de nervures (61, 62) sur leurs surfaces extérieures.

7. Seringue selon la revendication 1, caractérisée en ce que l'un au moins des deux ergots (63, 64) est effilé en direction de l'extrémité axiale de l'étrier (6).

8. Seringue selon la revendication 1, caractérisée en ce que les crémaillères (53, 54 ; 180, 190 ; 91, 92 ; 93, 94) sont formées sur un insert (5, 105) fixé au corps (10) de seringue.

9. Seringue selon la revendication 8, caractérisée en ce que l'insert (5) est formé d'un corps (51) demi-cylindrique terminé par une plaque d'extrémité (50) qui lui est perpendiculaire.

10. Seringue selon la revendication 9, caractérisée en ce que le corps (51) de l'insert (5) est séparé, par la nervure (52) longitudinale disposée sur sa paroi interne se terminant par la pointe incurvée (55) s'étendant dans un crevé (56), en deux parties portant chacune une série de languettes dont les extrémités dépassent de la paroi interne en direction de l'axe afin de confèrer audit corps (51) une section longitudinale en forme de crémaillères (53, 54).

11. Seringue selon la revendication 10, caractérisée en ce que la pointe incurvée (55) a son extrémité disposée longitudinalement à l'avant des languettes formant la crémaillère (53) autorisant la sortie du piston (2).

12. Seringue selon la revendication 1, caractérisée en ce que le piston (2) comporte deux portions (22, 23) reliées par une tige axiale cylindrique (24).

13. Seringue selon la revendication 12, caractérisée en ce que ladite tige axiale cylindrique (24) est séparée en deux parties par une liaison fragile (25).

14. Seringue selon la revendication 12, caractérisée en ce que le diamètre de ladite tige axiale (24) et sa longueur sont tels que l'étrier (6) puisse être positionné sur elle de manière à la chevaucher.

15. Seringue selon l'une quelconque des revendications 10 à 14, caractérisée en ce que la distance séparant axialement la face interne de la plaque d'extrémité (50) de l'insert (5) de l'extrémité de la pointe incurvée (55) est supérieure à la longueur de l'étrier (6) de sorte que lorsque le mouvement de sortie du piston est terminé, l'étrier (6) soit totalement disposé entre la plaque d'extrémité (50) et ladite pointe (55) s'étende axialement dans le prolongement des ergots (63, 64), et que, lors du mouvement de retour du piston (2) vers l'intérieur du corps (10) de la seringue, l'ergot (64) entre en contact avec ladite pointe incurvée (55) et entraîne l'étrier (6) en rotation pour amener les ergots (64, 63) dans l'axe des languettes formant la crémaillère (54) autorisant le retour du piston vers le fond du corps de seringue, sans que le piston soit entraîné en rotation.

16. Seringue selon la revendication 3, caractérisée en ce qu'elle porte une aiguille double constituée de deux aiguilles insérées l'une dans l'autre, une aiguille fine interne soudée à l'extrémité du corps de seringue et une aiguille amovible de diamètre supérieur positionnée autour de ladite aiguille fine de façon à pratiquer l'injection au patient avec l'aiguille fine.

## Claims

1. Syringe for administering a given number of injections comprising a body (10) in which a piston (2) is movable, of the kind comprising at least two racks (53, 54; 180, 190; 91, 92; 93, 94) formed by tongues and borne by the body (10), one (53, 180, 91, 93) of these racks authorizing outward motion of the piston (2) and the other (54, 190, 92, 94) authorizing the return thereof towards the bottom of the body (10), and two lugs borne by the piston (2) and driven in translation by the latter, each of said racks being capable of being shifted in a single direction in relation to a lug, characterized in that :
- said racks (53, 54; 180, 190; 91, 92; 93, 94) are fitted on to the body(10),
- said racks (53, 54; 180, 190; 91, 92; 93, 94) are separated by a rib (52, 170, 71, 72, 73),
- one lug (63) is intended to come into abutment against the tongues of a rack (53) in order to block all reverse motion of the piston (2) during filling of the syringe, the other lug (64) is intended to come into abutment against the tongues of the other rack (54) to prevent any withdrawal motion of the piston when the piston is pushed towards the bottom of the syringe,
- said lugs (63, 64) are borne by a yoke (6) which straddles the piston (2) and is rotatably mobile about the axis of said piston (2) so as to be driven in translation by the piston (2) while rotating freely in relation to the latter,
- a curved tip or oblique tongue (55, 81, 83) is formed at an end of the rib (52, 71, 73) so as to bring the lugs (63, 64) opposite the rack enabling the piston (2) to return towards the bottom of the syringe body.

2. Syringe as claimed in claim 1, characterized in that the rack (180) authorizing the outward motion of the piston (2) comprises at least one tongue which is oblique in relation to the axis of the syringe so as to enable it to be passed by the lugs (63, 64) of the yoke (6) during the outward motion of the piston, and to impose upon the yoke (6), during the inward motion of the piston, a rotating motion so as to position the lugs (63, 64) of the yoke (6) opposite the tongues of the second rack (190) in such a way that it will be possible to push in the piston when each of the oblique tongues of the rack have been passed over.

3. Syringe as claimed in claim 1, characterized in that it comprises several couples of alternate racks (91, 92; 93, 94), the first rack (91) allowing the piston (2) to move outwards from the body of the syringe, and the last rack (94) enabling the piston (2) to be driven back towards the bottom of the syringe body, and oblique tongues (81, 82, 83) situated at the end of each rack excepting the last rack to ensure the rotation of the yoke (6) so that the lugs (63, 64) cooperate with the next rack every time the direction of displacement of the piston is reversed.

4. Syringe as claimed in claim 3, characterized in that the racks (54; 190; 92; 94) having an even order include transversal tongues enabling a longitudinal motion of the piston (2) while preventing the reverse longitudinal motion of said piston (2), and at least one of the racks (53; 180; 91; 93) having an uneven order has oblique tongues inclined in relation to the axis of the syringe in such a manner to enable a longitudinal motion of the piston (2) in one direction while preventing a direct reverse motion of the piston (2) towards the bottom of the syringe body, said oblique tongues making the lug (64) which is cooperating with them have a circumferential motion taking it away from the axis of the rack during the reverse motion of the piston towards the bottom of the syringe body.

5. Syringe as claimed in claim 1, characterized in that the yoke (6) is of globally U-shaped cross section and bears said lugs (63, 64) on its outer side in the rounded portion of the "U" shape.

6. Syringe as claimed in claim 5, characterized in that the ends of said "U" shape are bordered by ribs (61, 62) on their outer surfaces.

7. Syringe as claimed in claim 1, characterized in that at least one of the two lugs (63, 64) is tapered in a direction of an axial end of the yoke (6).

8. Syringe as claimed in claim 1, characterized in that the racks (53, 54; 180, 190; 91, 92; 93, 94) are formed on an insert (5, 105), fitted onto the body (10) of the syringe.

9. Syringe as claimed in claim 8, characterized in that the insert (5) is made of a semi-cylindrical body (51) ended by an end plate (50) perpendicular to it.

10. Syringe as claimed in claim 9, characterized in that the body (51) of the insert (5) is separated, by the longitudinal rib (52) disposed on its inner wall ending in the curved tip (55) extending into a recess (56), into two parts each bearing a series of tongues whose ends protrude from the inner wall in the direction of the axis in order to confer upon said body (51) a longitudinal section in the shape of racks (53, 54).

11. Syringe as claimed in claim 10, characterized in that the curved tip (55) has its end disposed longitudinally to the front of the tongues forming the rack (53) authorizing outward motion of the piston (2).

12. Syringe as claimed in claim 1, characterized in that the piston (2) comprises two portions (22, 23) connected together by a cylindrical axial rod (24).

13. Syringe as claimed in claim 12, characterized in that said cylindrical axial rod (24) is separated into two parts by a fragile link (25).

14. Syringe as claimed in claim 12, characterized in that the diameter and length of said axial rod (24) are such that the yoke (6) can be positioned there upon in such a way as to straddle it.

15. Syringe as claimed in anyone of claims 10 to 14, characterized in that the distance axially separating the inner side of the end plate (50) of the insert (5) from the end of the curved tip (55) is greater than the length of the yoke (6) so that, when the outward pulling motion of the piston is completed, the yoke (6) is completely disposed between the end plate (50) and said tip (55) extends axially in the prolongation of the lugs (63,64), and that, during the return motion of the piston (2) towards the inside of the syringe body (10), the lug (64) comes into contact with said curved tip (55) and rotates the yoke (6) to bring the lugs (64,63) into the axis of the tongues forming the rack (54) authorizing the return motion of the piston towards the bottom of the syringe body, without the piston being rotated.

16. Syringe as claimed in claim 3, characterized in that it bears a double needle made of two needles inserted one in the other, a thin internal needle welded to the end of the syringe body and a removable needle of a larger diameter positioned around said thin needle so as to administer to the patient the injection with the thin needle.

## Patentansprüche

1. Spritze zum auf eine bestimmte Anzahl von Injektionen begrenzten Gebrauch, die aus einem Körper (10) gebildet ist, in dem sich ein Kolben (2) verschiebt, in der Bauart, die mindestens zwei aus Zungen geformte und durch den Körper (10) gestützte Rasterleisten (53, 54; 180, 190; 91, 92; 93, 94), wobei eine (53, 180, 91, 93) dieser Rasterleisten den Austritt des Kolbens (2) und die andere (54, 190, 92, 94) seine Rückkehr zum Boden des Körpers (1) der Spritze gestattet, und zwei vom Kolben (2) gestützte und von diesem bei einer Verschiebung mitgenommene Vorsprünge (63, 64) aufweist, wobei jede der Rasterleisten gegenüber einem Vorsprung eine Relativverlagerung in eine einzige Richtung haben kann,
dadurch gekennzeichnet, daß
die Rasterleisten (53, 54; 180, 190; 91, 92; 93, 94) am Körper (10) befestigt sind,
die Rasterleisten (53, 54; 180, 190; 91, 92; 93, 94) durch eine Rippe (52, 170, 71, 72, 73) geteilt werden,
einer (63) der Vorsprünge dazu bestimmt ist, sich an die Zungen einer Rasterleiste (53) anzulegen, um jegliche Gegenbewegung des Kolbens (2) während des Füllens der Spritze zu blockieren, wobei der andere Vorsprung (64) dazu bestimmt ist, sich an die Zungen der anderen Rasterleiste (54) anzulegen, um jegliche Rückzugsbewegung des Kolbens zu blockieren, wenn er zum Boden des Spritzenkörpers zurückgetrieben wird,
sich die Vorsprünge (63, 64) auf einen Bügel (6) stützen, der den Kolben (2) übergreift und um die Achse des Kolbens (2) derart drehbar ist, daß sie durch den Kolben (2) bei einer Verschiebung mitgenommen werden, obgleich sie diesem gegenüber frei drehbar sind,
eine gekrümmte Spitze oder eine schräge Zunge (55, 81, 83) am Ende der Rippe (52, 71, 73) ausgebildet ist, um die Vorsprünge (63, 64) bezüglich der Rasterleiste zu verstellen, die die Rückkehr des Kolbens (2) zum Boden des Körpers der Spritze gestattet.

2. Spritze gemäß Patentanspruch 1,
dadurch gekennzeichnet, daß
die den Austritt des Kolbens (2) gestattende Rasterleiste (180) mindestens eine zur Achse der Spritze schräge Zunge aufweist, wodurch ihre Überwindung durch die Vorsprünge (63, 64) des Bügels (6) beim Austritt des Kolbens gestattet ist und dem Bügel (6) beim Wiedereintritt des Kolbens eine Drehbewegung auferlegt wird, um die Vorsprünge (63, 64) des Bügels (6) bezüglich der Zungen der zweiten Rasterleiste (190) derart anzuordnen, daß nach Überwinden jeder der schrägen Zungen der Rasterleiste der Wiedereintritt des Kolbens gestattet ist.

3. Spritze gemäß Patentanspruch 1,
dadurch gekennzeichnet, daß
sie mehrere Paare wechselnder Rasterleisten (91, 92; 93, 94), wobei die erste Rasterleiste (91) den Austritt des Kolbens (2) aus dem Körper der Spritze und die letzte (94) die Rückkehr des Kolbens (2) zum Boden des Körpers der Spritze gestattet, und schräge Zungen (81, 82, 83) aufweist, die am Ende jeder außer der letzten Rasterleiste angebracht sind, um die Drehung des Bügels (6) derart sicherzustellen, daß die Vorsprünge (63, 64) mit der auf jede Richtungsänderung der Verlagerung des Kolbens folgenden Rasterleiste zusammenwirken.

4. Spritze gemäß Patentanspruch 3,
dadurch gekennzeichnet, daß
die geradzahligen Rasterleisten (54; 190; 92; 94) Querzungen aufweisen, die eine Längsverlagerung des Kolbens (2) bei Unterbindung einer entgegengesetzten Längsbewegung des Kolbens (2) gestatten, und wobei mindestens eine der ungeradzahligen Rasterleisten (53; 180; 91; 93) schräge Zungen aufweist, die gegenüber der Achse der Spritze derart geneigt sind, daß sie die Längsverlagerung des Kolbens (2) in eine Richtung gestatten und eine direkte Rückkehr des Kolbens (2) zum Boden des Spritzenkörpers unterbinden, wobei die schrägen Zungen den mit ihnen zusammenwirkenden Vorsprung (64) bei der Rückwärtsbewegung des Kolbens zum Boden des Spritzenkörpers so drängen, daß eine von der Achse der Rasterleiste beabstandete Umfangsbewegung bewirkt wird.

5. Spritze gemäß Patentanspruch 1,
dadurch gekennzeichnet, daß
der Bügel (6) einen im allgemeinen U-förmigen Querschnitt hat und die Vorsprünge (63, 64) auf seiner Außenfläche im abgerundeten Teil des U trägt.

6. Spritze gemäß Patentanspruch 5,
dadurch gekennzeichnet, daß
die Enden des U auf ihren Außenflächen von Rippen (61, 62) begrenzt sind.

7. Spritze gemäß Patentanspruch 1,
dadurch gekennzeichnet, daß
sich mindestens ein Vorsprung (63, 64) in Richtung des axialen Endes des Bügels (6) verjüngt.

8. Spritze gemäß Patentanspruch 1,
dadurch gekennzeichnet, daß
die Rasterleisten (53, 54; 180, 190; 91, 92; 93, 94) auf einem am Körper (10) der Spritze befestigten Einsatzteil (5, 105) ausgebildet sind.

9. Spritze gemäß Patentanspruch 8,
dadurch gekennzeichnet, daß
das Einsatzteil (5) aus einem halbzyindrischen Körper (51) gebildet ist, der mit einer zu ihm senkrechten Endplatte (50) abgeschlossen ist.

10. Spritze gemäß Patentanspruch 9,
dadurch gekennzeichnet, daß
der Körper (51) des Einsatzteiles (5) durch die an seiner Innenwand angeordnete Rippe (52) in Längsrichtung, die in der sich in eine Vertiefung (56) erstreckenden gekrümmten Spitze (55) endet, in zwei Teile unterteilt ist, wobei jeder eine Reihe von Zungen trägt, deren Enden aus der Innenwand in Richtung der Achse hinausragen, um dem Körper (51) einen Längsschnitt in Form von Rasterleisten (53, 54) zu geben.

11. Spritze gemäß Patentanspruch 10,
dadurch gekennzeichnet, daß
die gekrümmte Spitze (55) ihr Ende längs vor den Zungen angeordnet hat, die die den Austritt des Kolbens (2) gestattende Rasterleiste (53) bilden.

12. Spritze gemäß Patentanspruch 1,
dadurch gekennzeichnet, daß
der Kolben (2) zwei Abschnitte (22, 23) umfaßt, die durch einen zylindrischen, axialen Schaft (24) verbunden sind.

13. Spritze gemäß Patentanspruch 12,
dadurch gekennzeichnet, daß
der zylindrische axiale Schaft (24) durch ein zerbrechliches Verbindungsstück (25) in zwei Teile geteilt ist.

14. Spritze gemäß Patentanspruch 12,
dadurch gekennzeichnet, daß
der Durchmesser des axialen Schafts (24) und seine Länge dergestalt sind, daß der Bügel (6) auf ihm so positioniert sein kann, daß er ihn übergreift.

15. Spritze gemäß einem der Patentansprüche 10 bis 14,
dadurch gekennzeichnet, daß
die Entfernung, die die innere Fläche der Endplatte (50) des Einsatzes (5) axial vom Ende der gekrümmten Spitze (55) trennt, größer als die Länge des Bügels (6) ist, so daß, wenn die Austrittsbewegung des Kolbens beendet ist, der Bügel (6), der komplett zwischen der Endplatte (50) und der Spitze (55) angeordnet ist, sich axial in die Verlängerung der Vorsprünge (63, 64) erstreckt, und daß, bei der Rückwärtsbewegung des Kolbens (2) ins Innere des Spritzenkörpers (10), der Vorsprung (64) in Kontakt mit der gekrümmten Spitze (55) kommt und den Bügel (6) in Drehung versetzt, um die Vorsprünge (64, 63) in die Achse der Zungen zu bringen, die die Rasterleiste (54) bilden, die die Rückkehr des Kolbens zum Boden des Spritzenkörpers gestatten, ohne daß der Kolben in Drehung versetzt wird.

16. Spritze gemäß Patentanspruch 3,
dadurch gekennzeichnet, daß
sie eine doppelte Kanüle trägt, die aus zwei ineinander gesteckten Nadeln besteht, wobei eine feine Innennadel an das Ende des Körpers der Spritze angeschweißt ist und eine unbewegliche Nadel mit größerem Durchmesser um die feine Nadel herum so positioniert ist, daß die Injektion am Patienten mit der feinen Nadel ausgeführt wird.
